# EUROPEAN PATENT APPLICATION

(11) **EP 4 487 823 A2**
(43) Date of publication of application: **08.01.2025**
(21) Application number: 24215515.8
(22) Date of filing: 22.05.2013
(51) Int. Cl.: A61F 2/844

(54) **TRANSCATHETER PROSTHETIC VALVE FOR MITRAL OR TRICUSPID VALVE REPLACEMENT**

(62) Divisional of application: 19170261.2
(71) Applicant: Valcare Medical, Inc., Wilmington, Delaware 19801 (US)
(72) Inventor: Yellin, Nadav, 5259501 Ramat Gan (IL)
(74) Representative: Potter Clarkson

(57) **Abstract**

A prosthesis secures a replacement valve in a heart. The prosthesis includes a radially expandable inflow section and outflow section, and migration blocker rods. The inflow section has a tapered shape and is implanted within an atrium of a heart adjacent a native valve annulus. The outflow section couples to the inflow section, and is configured to be implanted through the native valve annulus and at least partially within a ventricle of the heart. The migration blocker rods extend circumferentially around at least a portion of the outflow section and hold native leaflets of the heart valve. In a contracted configuration, the prosthesis may be implanted through a catheter into the heart. In an expanded configuration, the tapered shape of the inflow section in the atrium cooperates with the migration blockers in the ventricle to hold the prosthesis against the native valve annulus.

## Description

### Technical Field

The present disclosure relates to implantable prosthetic devices. The disclosure is particularly useful in prosthetic devices implantable by catheter for the treatment of mitral or tricuspid regurgitation. The cause of the regurgitation can be either functional or degenerative or any other reason. Certain disclosed embodiments may be used for other valvular lesions as well.

### Background

Mitral Regurgitation is a valvular dysfunction that causes blood volume to flow during systolic (during left ventricular contraction) from the left ventricle to the left atrium in oppose to a healthy heart where this direction of flow is blocked by the mitral valve. The reverse flow during systolic causes pressure rise in the left atrium. Maintaining a normal cardiac output results in an increased left ventricle pressure.

Treating patients with MR or TR (mitral regurgitation or tricuspid regurgitation) could require valve replacement in order to reduce or eliminate the regurgitation. For many years the acceptable common treatment was surgical repair or replacement of the native valve during open heart surgery. In recent years, a trans vascular technique has been developed for introducing and implanting a prosthetic heart valve using a flexible catheter in a manner that is less invasive than open heart surgery.

In the trans vascular technique, the prosthetic is delivered to the target site (aortic valve, mitral valve, tricuspid valve, or other valve) through a catheter while the device is crimped to a low diameter shaft, and when it is located in the correct position it is expanded/ deployed to the functional size.

The advancing of the catheter to the target site can be through: (a) The vascular system where a catheter is advanced from the femoral vein/ artery, or any other blood vessel that allows access to the to the target site; (b) Trans apical where a catheter is advanced through a small incision made in the chest wall and then through the apex; or (c) Trans atrial where a catheter is advanced through a small incision made in the chest wall and then through the left or right atrium.

### Summary

A prosthesis secures a replacement valve in a heart. The prosthesis includes a radially expandable inflow section and outflow section, and migration blocker rods. The inflow section has a tapered shape and is implanted within an atrium of a heart adjacent a native valve annulus. The outflow section couples to the inflow section, and is configured to be implanted through the native valve annulus and at least partially within a ventricle of the heart. The migration blocker rods extend circumferentially around at least a portion of the outflow section and hold native leaflets of the heart valve. In a contracted configuration, the prosthesis may be implanted through a catheter into the heart. In an expanded configuration, the tapered shape of the inflow section in the atrium cooperates with the migration blockers in the ventricle to hold the prosthesis against the native valve annulus.

Additional aspects and advantages will be apparent from the following detailed description of preferred embodiments, which proceeds with reference to the accompanying drawings.

### Brief Description of the Drawings

Figure 1A illustrates a short axis view of a heart with four valves.
Figure 1B illustrates short axis view of mitral valve leaflets.
Figure 2A illustrates a three chambers view (long axis) of the heart.
Figure 2B illustrates a two chambers view (long axis) of the heart.
Figure 3 is an isometric view of a stent configured for placement in a native mitral or tricuspid valve according to one embodiment.
Figure 4 is a front view of the stent shown in figure 3.
Figure 5A is a top view of a stent with an elliptical inflow and circular outflow according to one embodiment.
Figure 5B is an isometric view of a stent with an elliptical inflow and a circular outflow according to one embodiment.
Figure 5C is an isometric view of a stent with a circular inflow and a circular outflow according to one embodiment.
Figure 5D is a top view of a stent with a circular inflow and a circular outflow according to one embodiment.
Figure 5E is an isometric view of a stent with an elliptical inflow and an elliptical outflow according to one embodiment.
Figure 5F is a top view of a stent with an elliptical inflow and an elliptical outflow according to one embodiment.
Figure 5G is an isometric view of a stent with a circular inflow and an elliptical outflow according to one embodiment.
Figure 5H is a top view of a stent with a circular inflow and an elliptical outflow according to one embodiment.
Figure 6A is a front view of a stent having an outflow with one row of struts according to one embodiment.
Figure 6B is a front view of a stent having an outflow with two rows of struts according to one embodiment.
Figure 7A is a front view of migration blocking rods of a stent according to one embodiment.
Figure 7B is a side view of the migration blocking rods of the stent shown in figure 7A.
Figure 7C is a bottom view of the migration blocking rods of the stent shown in figure 7A.
Figure 8A is a front view of migration blocking rods with ends close to each other according to one embodiment.
Figure 8B is a front view of migration blocking rods with ends far from each other according to one embodiment.
Figure 9A is a front view of a stent illustrating a curvature of the inflow (high profile inflow) according to one embodiment.
Figure 9B is a front view of a stent illustrating a curvature of the inflow (low profile inflow) according to one embodiment.
Figure 10A is an isometric view of a stent including migration rods with a leading mechanism at the distal end according to one embodiment.
Figure 10B is an enlarged view of the leading mechanism at the distal end of the migration blocking rods shown in figure 10A.
Figure 11A is a front view of a stent including a migration locking mechanism with snapping according to one embodiment.
Figure 11B is an enlarged view of the migration locking mechanism with snapping shown in figure 11A.
Figure 11C is an isometric enlarged view of the migration locking mechanism with snapping shown in figure 11A.
Figure 12A is an isometric view of a stent including barbs extending from the inflow section according to one embodiment.
Figure 12B is an enlarged view of a barb shown in figure 12A.
Figure 13A is an isometric view of a stent including separate inflow and outflow sections according to one embodiment.
Figure 13B is an isometric view of the separated inflow section shown in figure 13A.
Figure 13C is an isometric view of the separated outflow section shown in figure 13A.
Figure 13D is an enlarged isometric view of a connection area of the inflow and outflow sections shown in figure 13A.
Figure 14A illustrates a stent inside a heart in a three chamber view according to one embodiment.
Figure 14B illustrates a stent inside a heart in a three chamber view according to one embodiment.
Figure 15 is a short axis view of a heart with a stent implanted therein according to one embodiment.
Figure 16A is an enlarged view of a barb shown in figure 16B according to one embodiment.
Figure 16B illustrates a locking mechanism between the migration blocker rods and the inlet according to one embodiment.
Figure 17 is a detailed cross-section view of migration blocker rods passing through the chordae according to one embodiment.
Figure 18 is a detailed cross-section view of a stent inside a heart, from a septal lateral perspective, according to one embodiment.
Figures 19A, 19B, 20, and 21 show an example of a trans atrial approach for trans catheter implantation of a stent in the mitral position according to one embodiment.
Figures 22A, 22B, 23, and 24 show an example of a trans apical approach for trans catheter implantation of a stent in the mitral position according to one embodiment.

### Detailed Description of Preferred Embodiments

When used the singular form "a", "an", "the" refers to one or more than one, unless the context clearly dictates otherwise.

As used herein, the term "includes" means "compromise" for example, a device that includes or compromises A and B contains A and B but can optionally contain C or other components other than A and B. A device that includes or compromises A and B may contain A or B, or A and B, and optionally one or more other components such as C.

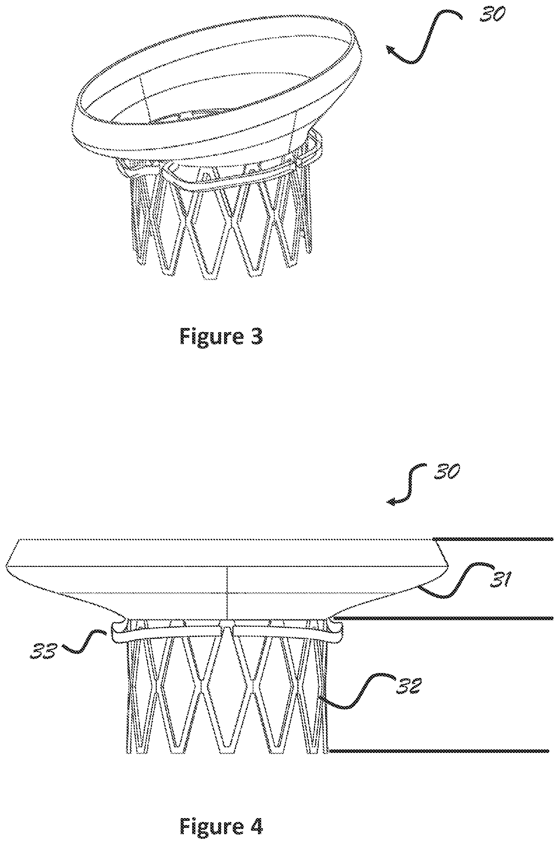

When the words "stent" and "frame" are used they refer to the same element (e.g., see stent 30 in figure 3).

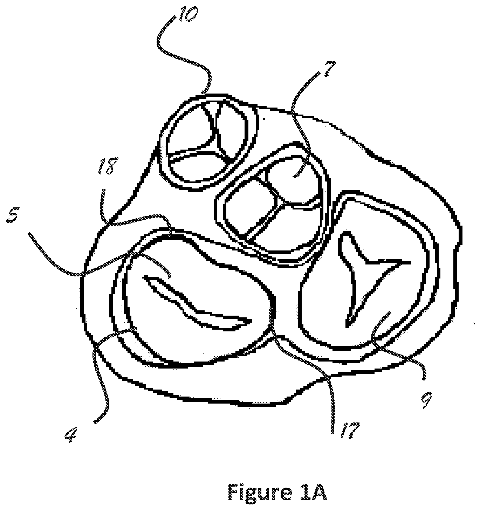

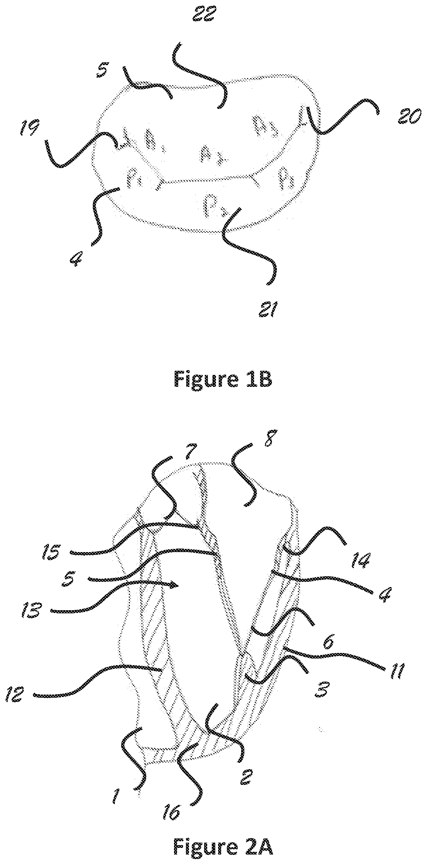

Figure 1A shows a short axis section of the four valves in a heart. The aortic valve 7, pulmonary valve 10, tricuspid valve 9, and mitral valve with anterior leaflet 5 and posterior leaflet 4. In figure 1B, there is an illustration of the mitral valve with posterior leaflet 4 sectioned into P1, P2, P3 and anterior leaflet 5 sectioned into A1, A2, and A3. These sectioning methods are common knowledge and acceptable among those skilled in the art. Figure 1B also shows a commissure 19 between A1 and P1 and a commissure 20 is the commissure between A3 and P3.

Figure 2A is a three chamber view (long axis) of the heart. In this view, the left atrium 8, left ventricle 2, and right ventricle 1 are shown. The aortic valve 7 is at the end of the left ventricle outflow tract (LVOT) 13. The mitral valve apparatus with mitral leaflets includes anterior leaflet 5 and posterior leaflet 4 attached to the chordae tandea 6 and papillary muscles 3. This view is a section of the mitral valve through the A2 (shown as area 22 in figure 1B) and P2 (shown as area 21 in figure 1B) areas of the mitral leaflets.

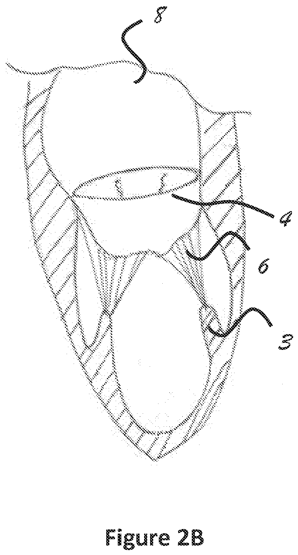

Figure 2B is a two chamber view (long axis) of the heart. In this view the left atrium 8 and left ventricle 2 are shown. The mitral valve apparatus includes the posterior mitral leaflet 4 attached to the chordae tandea 6 and papillary muscles 3. This view is a section of the mitral valve through the commissures 19 and 20 of the mitral leaflets.

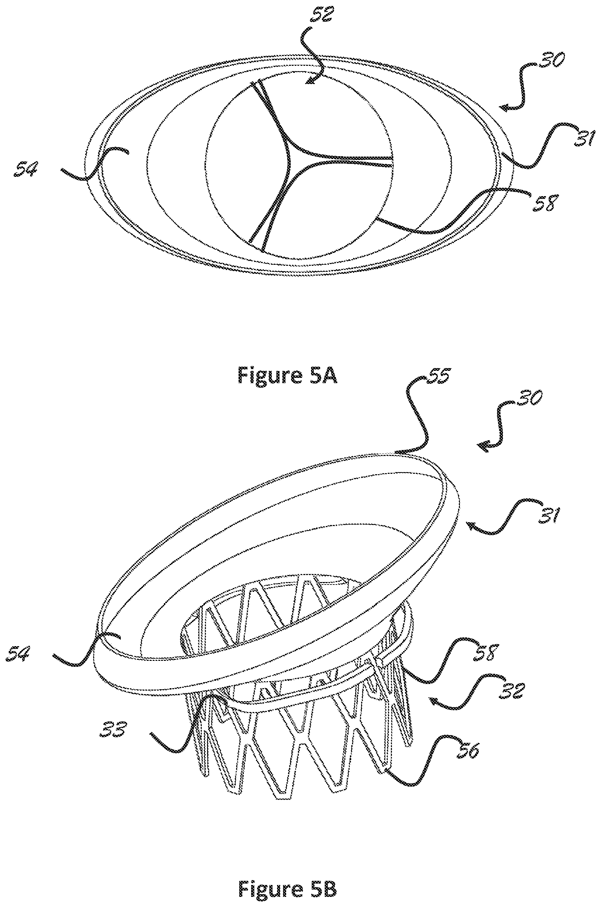

Figure 3 is a perspective view of a stent 30 configured for placement in a native mitral or tricuspid valve. The stent 30 in figure 4 is a front view of the stent 30 shown in figure 3. In this embodiment, the stent 30 includes an upper section 31 (also referred to herein as "inflow section" 31) having an enlarged diameter (circumference) or flared end that tapers into a lower section 32 (also referred to herein as "outflow section" 32) of the frame having a reduced diameter (circumference). The upper section 31 and/or the lower section 32 may have different shape than circular. The stent 30 might have any combination of shapes and figures 5A-5H are only examples of the different shapes possible and other may apply as well. Migration blocker rods 33 shown in figures 3 and 4 are separated rods, which after deployment lean against the native annulus and prevent migration of the stent into the atrium 8 shown in figure 2A. The migration blocker rods 33 can be in different lengths with different ends and additional features can be added on them, such as: A. leading mechanism to ensure connectivity, after deployment, between different migration blocker rods; B. locking mechanism between the rods; C. barbs to prevent rocking; and D. features that lock the migration blocker rods against the upper section 31 of the frame 30.

Inside the stent assembly a prosthetic valve (not shown) might be added. The valve can be either bi-leaflet or tri-leaflet as long as it performs as required and can be made out of any tissue, polymer, or other material, as long as it is biocompatible. The stent 30 can be self-expanding stent made of a shape memory material such as, for example, Nitinol. It can be cut of tube, sheet, or/and a pattern that allows crimping and expanding like braided wires or different technique that attaches wires as long as it performs well.

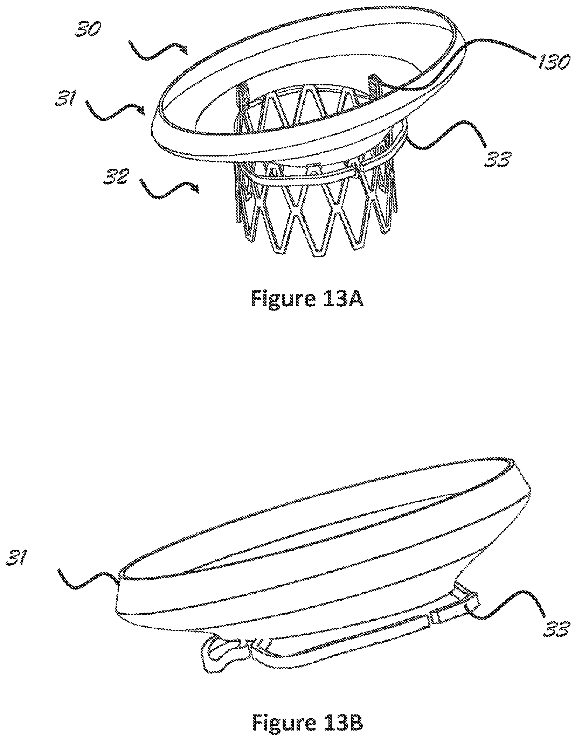

In other embodiments, the stent 30 can be a combination of a self-expanding stent and a balloon expandable stent. For example, figures 13A-13D demonstrate an upper section 31 including a shape memory alloy that functions as a self-expandable frame, and a lower section 32 including a balloon expandable stent that requires balloon inflation for final deployment. The two sections can be attached in any way. For example, welding, mechanical attachment (as shown in figures 13A-13D), and/or additional features that attach them are only some of the ways to attach the two sections of the stent assembly.

The raw material of the stent 30 can be metal or any kind that is biocompatible. The stent 30 may include a combination of two or more different materials. For example, one part from stainless steel 316/316L and another part from Nitinol. Other materials such as cobalt chrome are only examples, and other materials can be used as well.

The design of the frame 30, either if it is from one part or more, is configured to allow crimping the prosthesis into a low profile shaft (equal or under 13 mm outer diameter (OD)). Patterns that allow this are known and crisscross patterns as shown for example in figures 3 and 4 for the outflow section 32 or braided stents are two examples and other may be applied as well.

The migration blocker rods 33 of the stent 30 lean against the native annulus of the tricuspid or mitral valve, in general. When used in the mitral position, the migration blocker rods 33 may lean, in specific, against the mitral groove 14 shown in figure 2A in the posterior side and against the left fibrous trigon 18 and the right fibrous trigon 17 in the anterior side shown in figure 1A.

On the atrium side, the flared upper section 31 prevents any migration of the stent 30 into the ventricle 1 or 2 shown in figure 2A and helps provide sealing between the stent and the native apparatus by verifying good intimate contact and correlation between the inflow section geometry and the native shape of the mitral annulus and left atrium.

The combination of the migration blocker rods 33 from the ventricle side of the native annulus and the upper section 31 flared stent from the atrium side of the annulus create a clamping effect on the annulus and provide a positive axial anchoring of the stent 30 to its target site.

For the upper section 31, according to certain embodiments, an elliptical shape allows reducing the inflow section projection and therefore reduces the area that faces high pressure during systole. This feature reduces the axial forces that the prosthesis faces and needs to be anchored against. At the same time, an elliptical shape assures continuous contact between the upper section 31 and the atrium and prevents any para valvular leakage (PVL). Any other shape that will at the same time prevent PVL and minimize the projection of the inflow is desired.

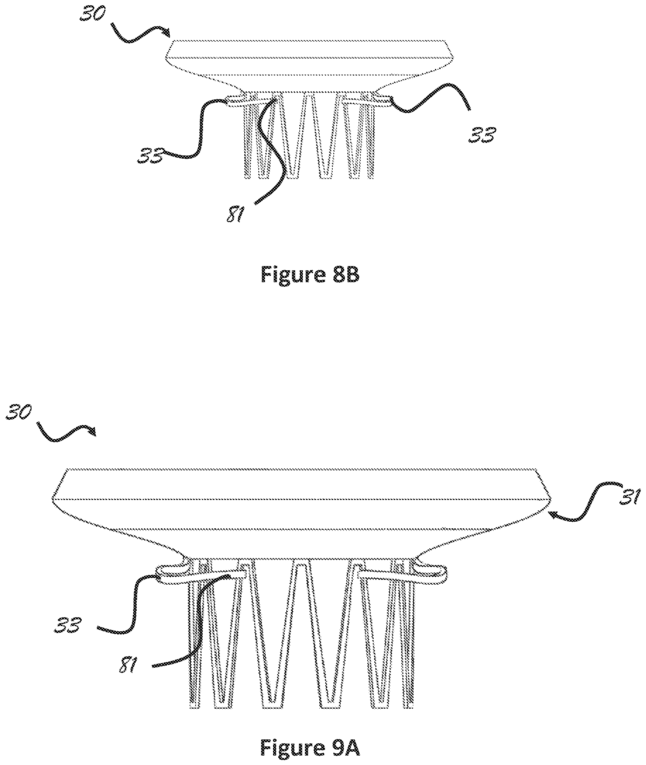

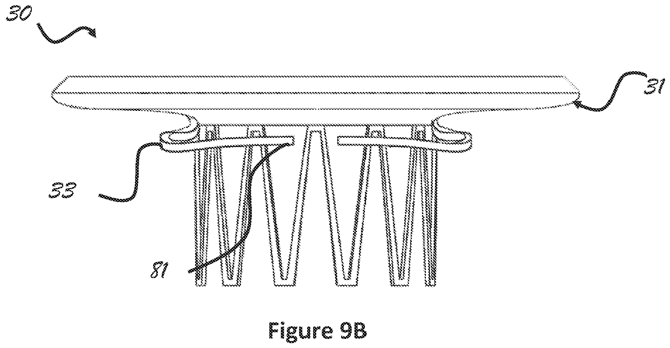

The curvature that defines the transition zone and/or the inflow section profile may be configured to increase or decrease the clamping effect between migration blocker rods 33 and the inflow section 31. Figures 9A and 9B show two examples and any other curvature that allows the upper section to be fixated in the atrium and the migration blocker rods to stay under the native annulus in the ventricle is acceptable.

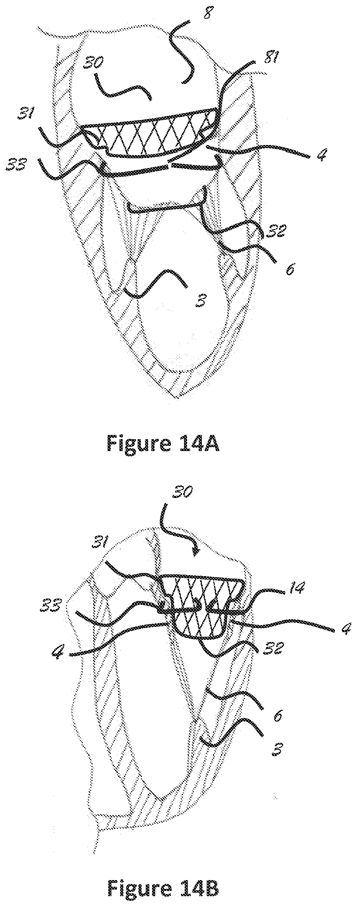

In the area of connection between the upper section 31 and lower section 32 of the stent 30 are attached migration blocker rods 33 which prevent from the valve from migrating into the left atrium. The migration blocker rods 33 go in between the chordae under the native commissures 19 and 20 shown in figure 1B and leans against the mitral annulus from behind the native leaflets. Figures 14A, 14B, 15, 16A, 16B, and 17 show the extraction of the migration blocker rods from the stent, passing through the chordae and turning around the native leaflets. At the final position, the rods 33 lean against the native annulus.

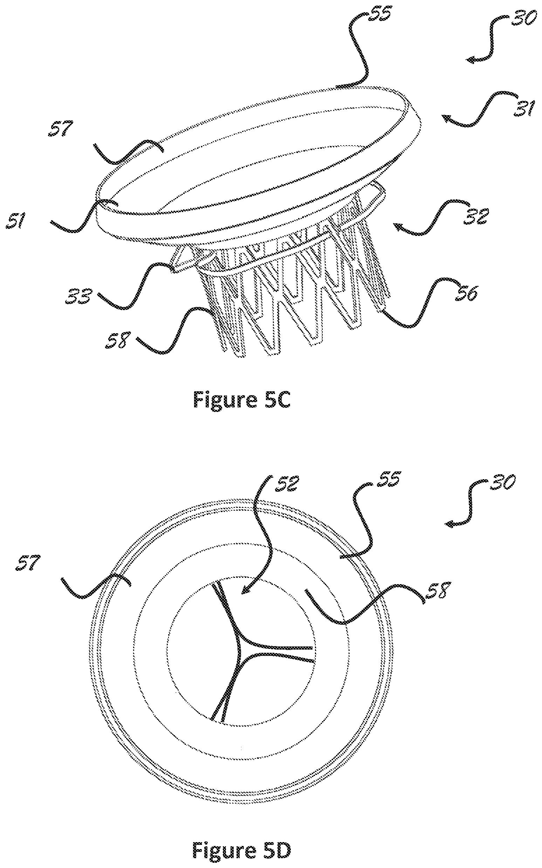

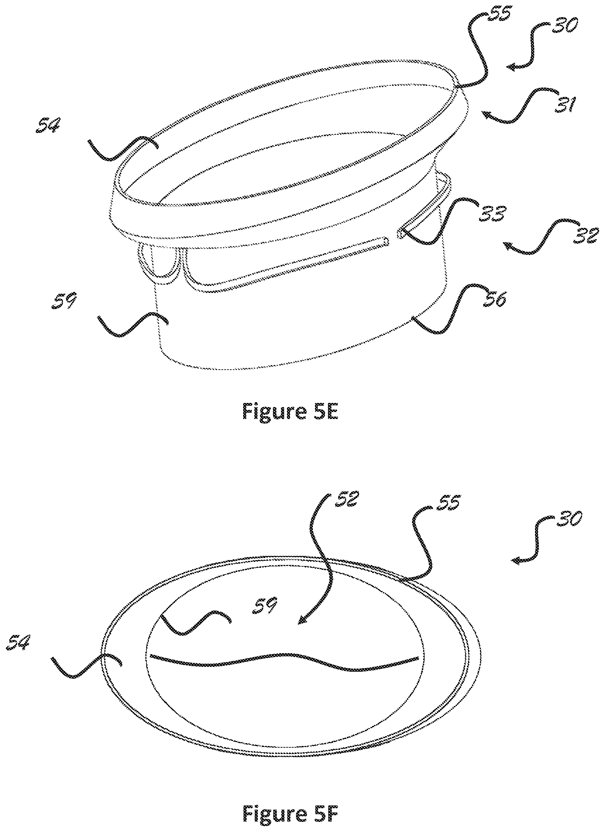

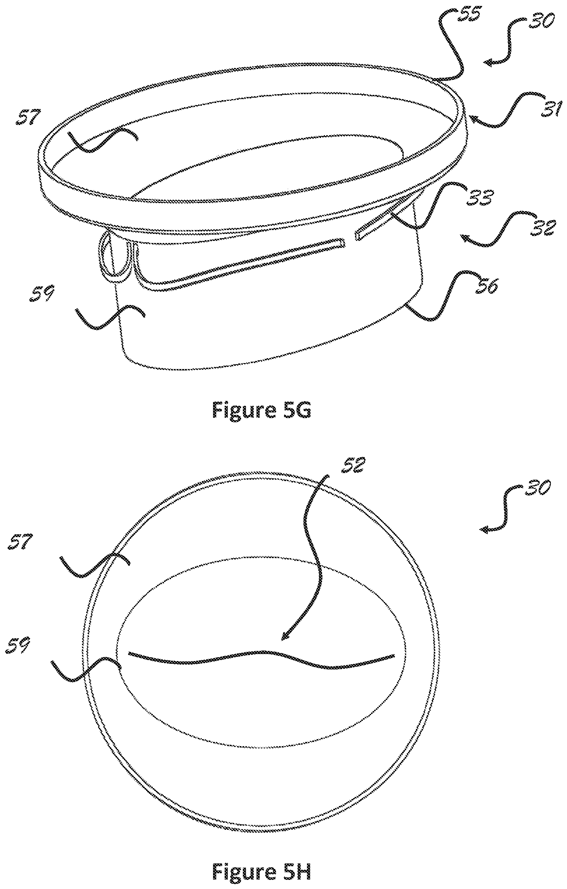

Figures 5A-5H represents different combinations of the inflow and outflow profiles. The inflow profile in the illustrated embodiments can be either circular 57 (as shown in figures 5C, 5D, 5G, and 5H) or elliptical 54 (as shown in figure 5A, 5B, 5E, and 5F), or any other shape that fits the native anatomy of the atrium. The outflow profile can be either circular 58 (as shown in figures 5A, 5B, 5C and 5D) or elliptical 59 (as shown in figures 5E, 5F, 5G and 5H), or any other shape that fits to withhold a prosthetic valve inside, either bi leaflet or tri leaflet. Figures 5A-5H illustrate, by way of example, only four combinations out of many possible of the options for the design of the stent 30.

In figures 5A-5H, the circumference of the inflow section 31 and its upper end 55 can vary between about 225 mm to 90 mm. This large variation is due to the target population of the device, which some have a very large atrium. The circumference of the outflow section 32 and its lower end 56 can vary between about 110 mm to 60 mm. This variation is to allow different sizes of valves inside the outflow according to the acceptable standards, if they exist, for the mitral and tricuspid position. The height of the stent may vary between about 20 mm to 60 mm, as long as it doesn't injure the left ventricle walls by the lower section 32 and lower end 56 and doesn't interfere with the flow from the pulmonary veins and/or cause any risk relatively to the left appendage. The valve 52 (shown in figures 5A, 5C, 5F, and 5H) can be either bi-leaflet or tri-leaflet as long as it performs as required and can be made out of any tissue, polymer, or other material as long as it is biocompatible. The stent 30 can be a self-expanding stent made of a shape memory material such as, for example, Nitinol. It can be cut of tube, sheet, or/and a pattern that allows crimping and expanding like braided wires or a different technique that attaches wires as long as it performs well.

In figures 5A and 5D, an illustrated tri leaflet valve 52 is mounted in the circular outflow section 32. The valve 52 is configured such that the flow of blood goes substantially only in one direction and that substantially no back flow will occur through the valve according to the acceptable standards.

The valve 52 can be composed from biological tissue such as pericardium or alternatively from a polymer, fabric, etc.

In other embodiments, such as 5F and 5H, the valve 52 in the outflow section 32 can be bi leaflet.

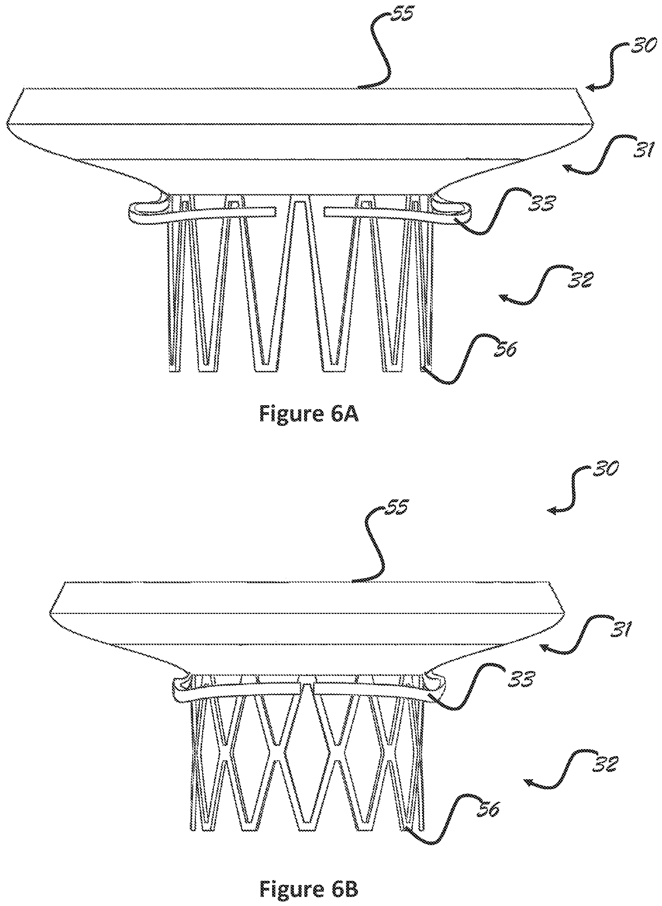

In figures 6A and 6B, there is a front view of the stent 30 according to certain embodiments. It is illustrated as an example that the stent 30 can have any number of rows of struts (illustrated as "V" shaped structural supports), as long as the struts allow crimping into a catheter and deployment to the final configuration. The outflow section 32 can have either 1 (one) row of struts or more. In the illustrated embodiments, there is an example of an outflow section 32 with 1 (one) row of struts in figure 6A, and an embodiment of an outflow section 32 with 2 (two) rows of struts in figure 6B. This is not limiting and more rows can be added. In certain embodiments, the inflow section 31 also includes expandable struts. For example, the inflow section 31 may be designed in a similar manner as that of the outflow section 32 with a criss-cross pattern and/or any number of rows of struts, as long as the expandable struts allow crimping and expanding of the inflow section 31 to its different configurations.

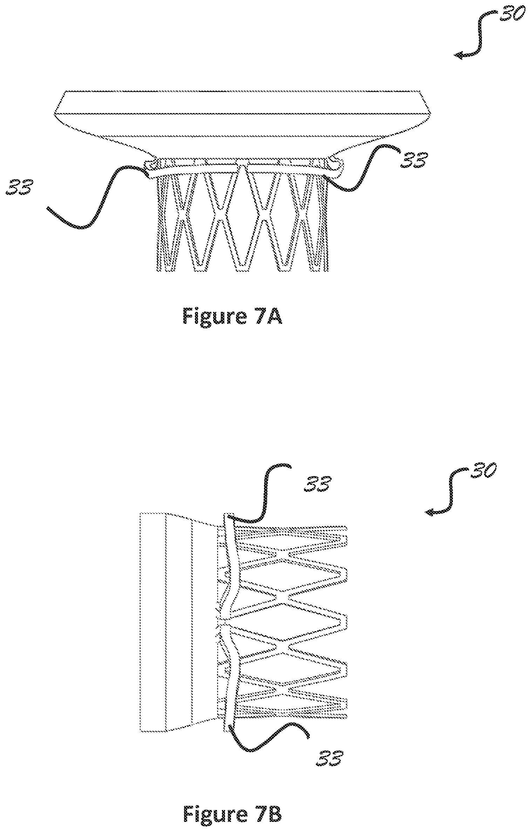

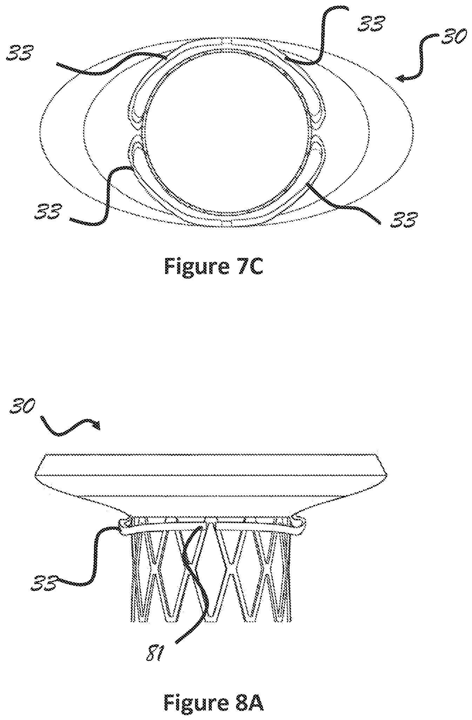

Figures 7A, 7B, and 7C illustrate the migration blocker rods 33 from three different views. Figure 7A illustrates the migration blocker rods 33 in stent 30 from a front view, figure 7B illustrates the migration blocker rods 33 in stent 30 from a side view, and figure 7C illustrates the migration blocker rods 33 in stent 30 from a bottom view. The rods 33 are configured to be attached to the stent 30 either to the inflow section 31 or to the outflow section 32 at the area where these sections are attached to each other, and to provide axial fixation of the stent 30 at the target site.

The migration blocker rods 33 around the posterior leaflet 4 are configured to lean against the mitral groove 14 and prevent any migration and axial movement in the posterior side.

The migration blocker rods 33 around the anterior leaflet 5 are configured to lean against the left and right fibrous trigons 17 and 18 and prevent any migration and axial movement in the anterior side.

There are one, two, or more migration blocker rods 33 around the posterior leaflet 4. There are another one, two, or more migration blocker rods 33 around the anterior leaflet 5. The quantity of the migration blockers can vary from two to multiple rods and in the certain illustrated embodiments there are four of them only for visualization and as example. In other embodiments, the quantity of migration blocker rods 33 can be any number from two to eighteen.

The migration blocker rods 33 can be ended separated from one another, can meet each other behind the leaflets 4 and 5, may include a leading mechanism behind the leaflet to ensure the attachment of the rods to one another and may include a locking mechanism that prevents them from separating after deployment.

The migration blocker rods 33 can be in different lengths with different ends 81 and additional features can be added on them. The end 81 of the migration blocker rods 33 can be seen in figures 8A and 8B. It can be seen that the distance between them can vary from zero, at minimum (they can touch each other), to, at maximum, half the circumference of the outflow section. In the later, the length of the rods 33 is very short and the point of leaning against the annulus is under the commissures 19 and 20 in figure 1B.

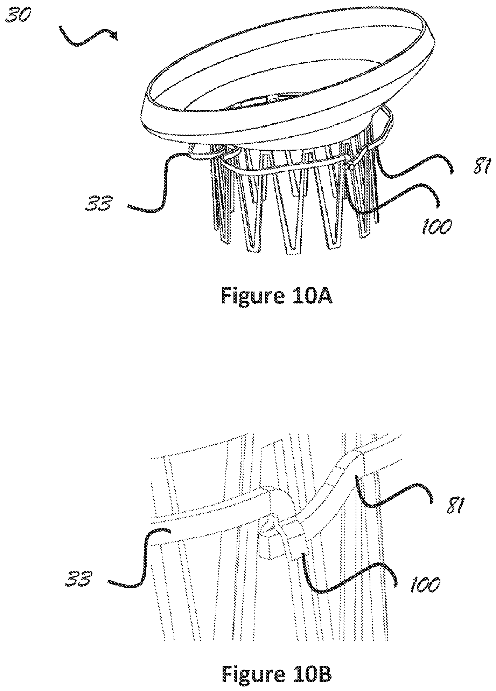

In figures 10A and 10B, there is a leading mechanism 100 at the end 81 of the migration blocker rods 33 that allows connecting two migration blocker rods 33 that come from opposite commissures 19 and 20. The leading mechanism 100 allows two different migration blocker rods 33 to meet and attach to each other. Due to the nature of beating heart procedures and no direct visualization (only through X-ray and ultrasound), it may be useful to have such a mechanism 100 that allows leading one rod 33 into the other to assure that the two can be connected. The illustrated mechanism 100 is only one example but others can be designed and might include wire, suture, metallic, and/or plastic members, etc.

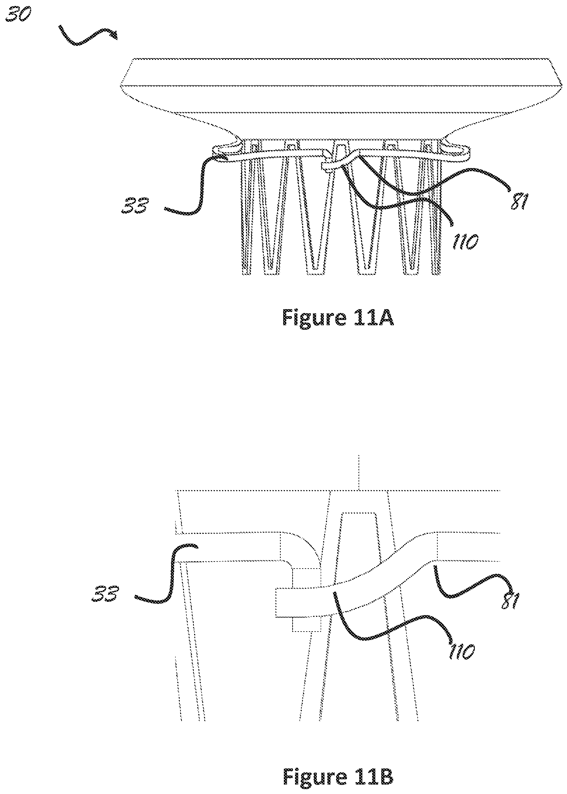

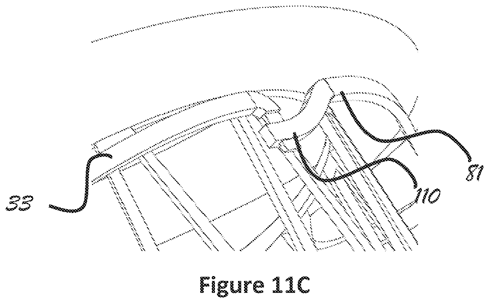

In figures 11A, 11B, and 11C, there is a snapping mechanism 110 at the end 81 of the migration blocker rods 33 that allows connecting two migration blocker rods 33 that come from opposite commissures 19 and 20 and lock them one into the other. Once two migration blocker rods 33 are attached and locked the stent is firmly secured in place and the rods 33 can't be crimped back to the crimped configuration unless the snap mechanism 110 is released. The snap illustrated in figures 11A, 11B, and 11C is one example for such mechanism and others with additional members as metallic and/or plastic parts, wire, suture can be added.

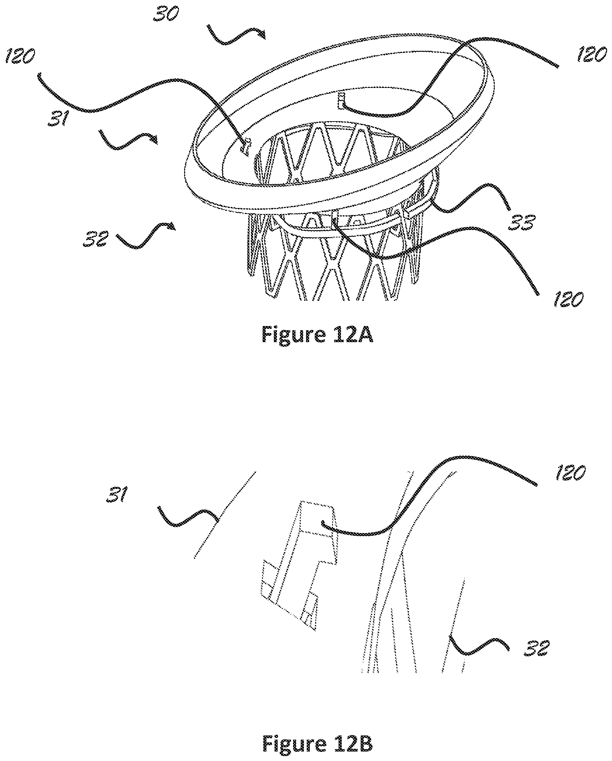

Figures 12A and 12B illustrate migration blocker rods 33 that include barbs 120 configured to penetrate the mitral annulus from the ventricle side and ensure no relative movement between the frame 30 and the mitral annulus. The barbs 120 that penetrated the mitral annulus can be locked into the inflow section of the frame from the atrium side or locked into an additional ring. Figure 12B is a zoom on the isometric view of a barb that is part of a migration blocker rod 33 that penetrated through the annulus into the inflow section 31.

The migration blocker rods 33 can be cut from the same tube and heat treated to the final shape. The migration blocker rods 33 can be cut from different tube and be attached to the main frame differently using a direct attachment such as welding or with additional members such as sutures, metallic parts, etc. The migration blocker rods 33 can be crimped distally to the main frame, proximally to the main frame and on top of it. The migration blocker rods 33 might be covered with a fabric, soft tissue, and/or polymer to prevent any damage to the annulus apparatus.

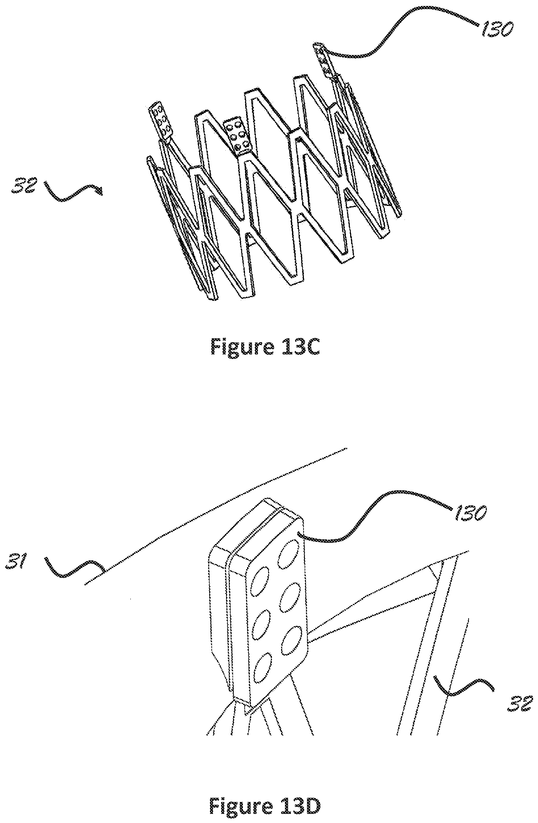

Figures 13A, 13B, 13C and 13D illustrate a stent 30 that includes two different sections. The inflow section 31 is a self-expanding stent made from a shape memory alloy and functions as a self-expandable frame, and the lower section 32 is a balloon expandable stent that requires balloon inflation for final deployment.

Figure 13A is an isometric view of the two sections attached together through an attachment member 130. The attachment member 130 can be part of the inflow section 31, outflow section 32, both the inflow section 31 and the outflow section 32, or/and as an additional member.

In figure 13B illustrates an example of an inflow section 31 made out of shape memory alloy where the migration blocker rods 33 are part of it. For example, inflow section 31 and the migration blocker rods 33 may be formed from the same piece of shape memory material. In other embodiments of the inflow section 31, the migration blocker rods 33 can be omitted, or designed differently. In addition, or in other embodiments of the inflow section 31, an attachment feature for connecting to the outflow section 32 can be added. An example of such a feature is a metallic flange that is cut of the frame and illustrated in the attached embodiments as attachment member 130.

Figure 13C illustrates an example of an outflow section 32 made out of an alloy such as stainless steel (StSt), such as StSt 316/ StSt 316L. In other embodiments, the outflow section 32 can be made out of self-expandable alloy such as shape memory alloy and might include the migration blocker rods 33. In addition, or in other embodiments of the outflow section 32, an attachment feature for connecting to the inflow section 31 can be added. An example of such a feature is a metallic flange that is cut of the frame and illustrated in the attached embodiments as attachment member 130.

Figure illustrates an enlarged view of the attachment feature 130 between the inflow section 31 and the outflow section 32. In this embodiment, the attachment feature 130 includes two metallic flanges. One is part of the inflow section 31 and one is part of the outflow section 32. The two flanges can be attached together by snapping one to another, suturing, them together, or any other attachment method.

Figure 14A and 14B illustrate how the stent 30 may be positioned in the mitral valve. In figure 14A, the section of the heart illustrates a two chamber view and the cross-section of the drawing passes through the mitral valve commissures. It can be seen that the stent 30 is behind the posterior leaflet 4, the migration blocker rods 33 pop out from the commissures 19 and 20, and the end 81 of the migration blocker rods 33 is in the P2 section of the leaflet (area 21 in figure 1 B). In figure 14B, the section of the heart illustrates a three chamber view and the cross-section of the drawing passes through the A2 and P2 (areas 21 and 22 in figure 1B) of the native valve. It can be seen that the stent 30 is between the posterior leaflet 4 and anterior leaflet 5, the migration blocker rods 33 pop out from the commissures area, and the end 81 of the migration blocker rods 33 is located in the posterior side under the mitral groove 14 and under the left and right fibrous trigons (18 and 17 in figure 1A) in the anterior side.

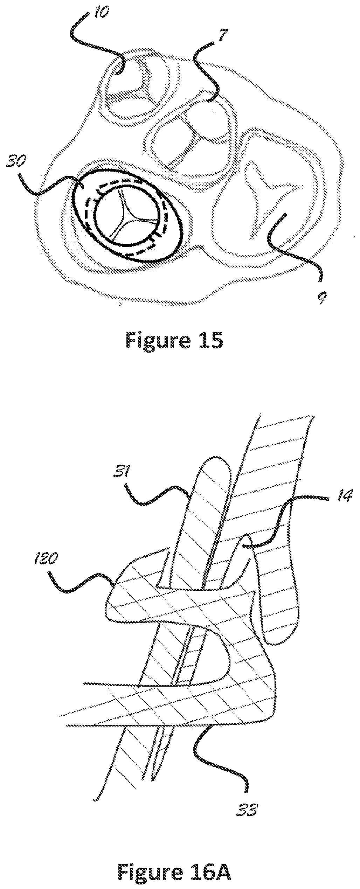

Figure 15 illustrates the stent 30 in the mitral valve from a short axis view from the atrial side. The migration blocker rods 33 are located in the ventricle side under the mitral leaflets.

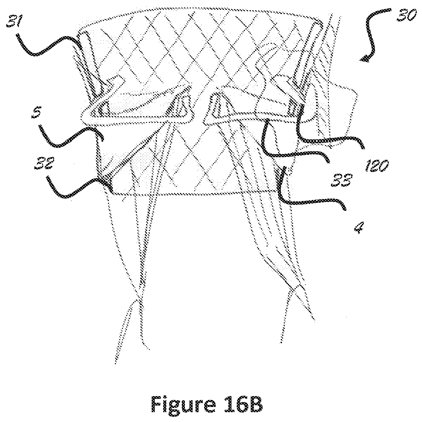

Figures 16A and 16B illustrate an additional feature that can be added to the migration blocker rods 33. The barbs 120 are part of the migration blocker rods 33 and designed in a way that after deployment they penetrate the mitral annulus and/ or mitral leaflets and anchor the stent to the annulus. The barbs 120 can be integral part of the migration blocker rods 33 or additional member that is assembled on the barbs. The barbs 120 may be configured so that they have an opposite member or feature in the inflow section 31 in a way that after crossing the tissue they lock into the inflow section.

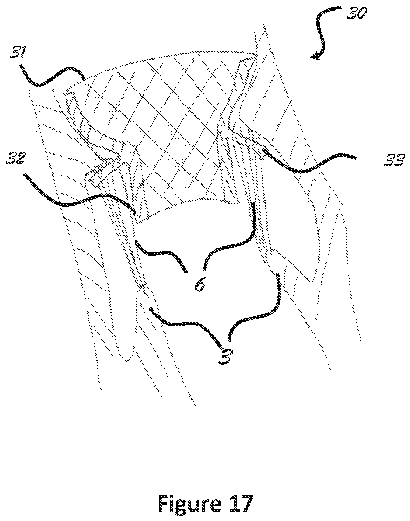

Figure 17 is an additional illustration that shows how the migration blocker rods 33 pass between the chordae tandea 6 in the commissures 19 and 20.

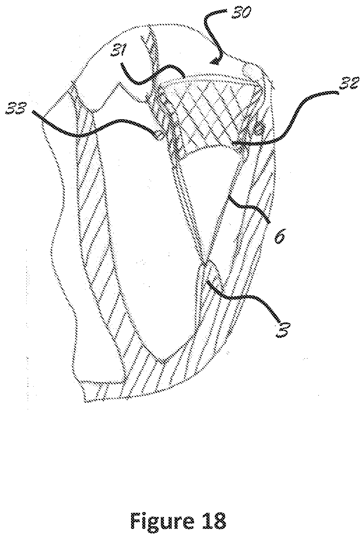

Figure 18 is an additional drawing illustrating how the migration blocker rod 33 leans against the mitral groove 14 in the posterior side and the left and right fibrous trigons on the anterior side.

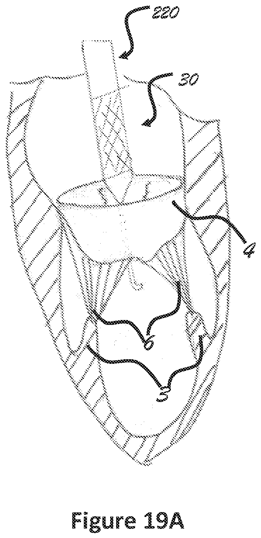

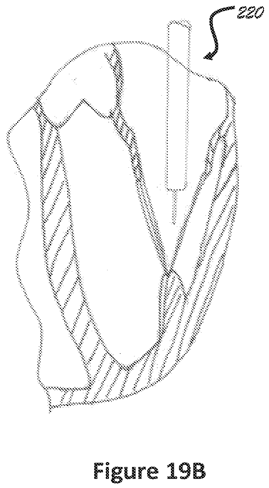

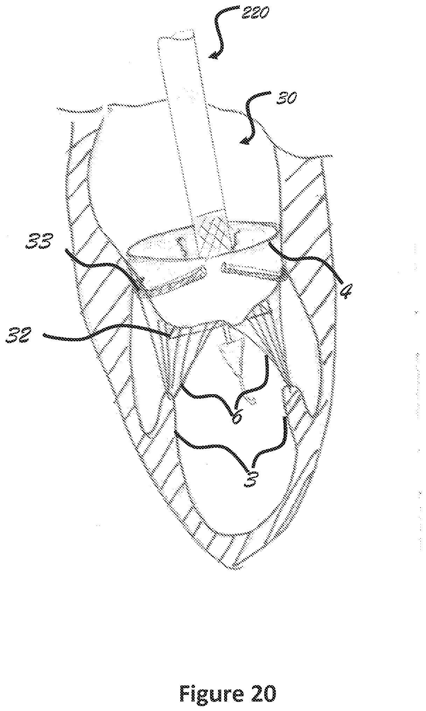

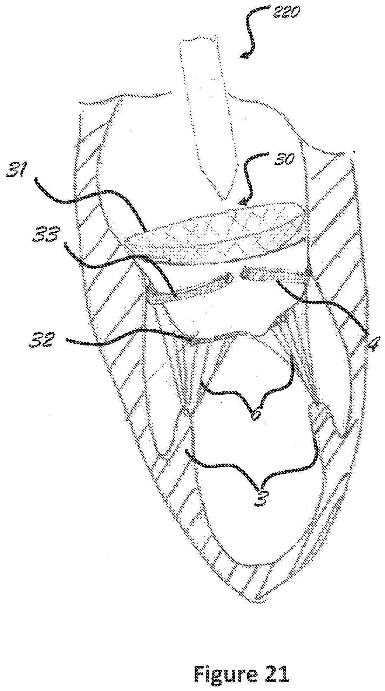

Figures 19A, 19B, 20, and 21 show an example of a trans atrial approach for trans catheter implantation in the mitral position. In figures 19A and 19B, the catheter is advanced through the left atrium 8 and then through the native mitral valve to the left ventricle. The stent 30 in this figure is crimped into the catheter shaft 220. The migration blocker rods are as well crimped in the shaft 220 and can be crimped distally toward the apex 16, proximally toward the entering point to the left atrium, or on top of the main frame 30. Figure 20 shows the deployment of the stent 30. The migration blocker rods 33 pass through the chordae 6 under the native commissures and circle the native leaflets. The migration blocker rods 33 are configured, in certain embodiments, to bypass or encircle the native leaflets without clamping them to the main frame 30. Then, a completion of the deployment results in clamping the native annulus and allowing the rods 33 to prevent migration and rocking. Figure 21 shows that the catheter 220 is withdrawn backwards after completion of the deployment.

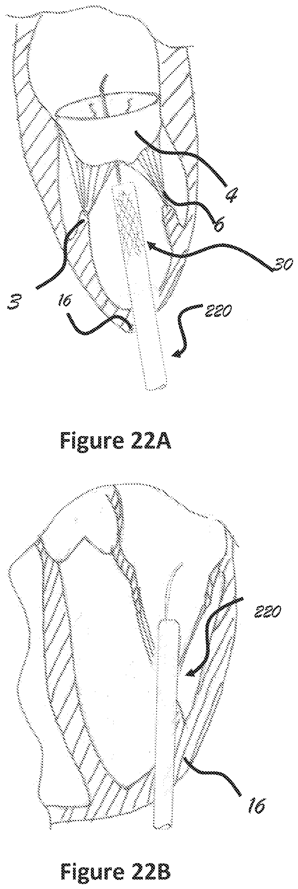

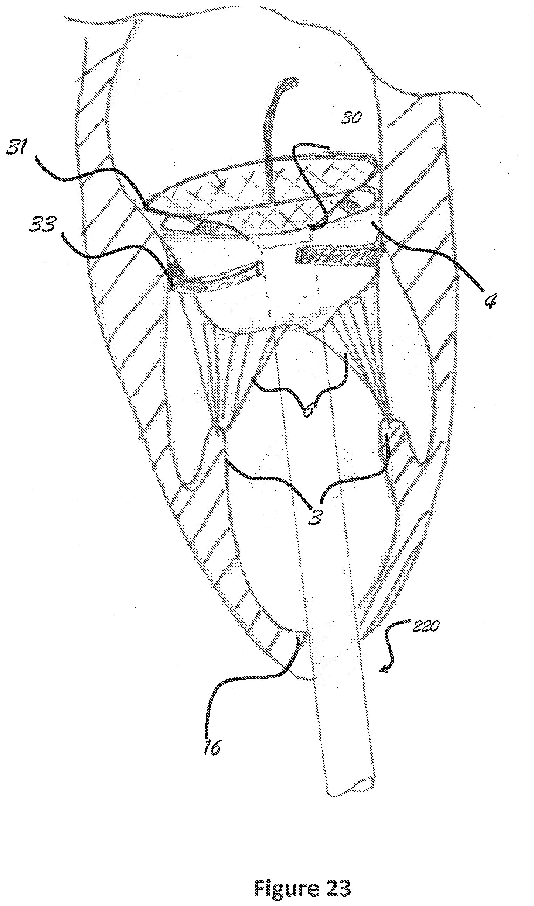

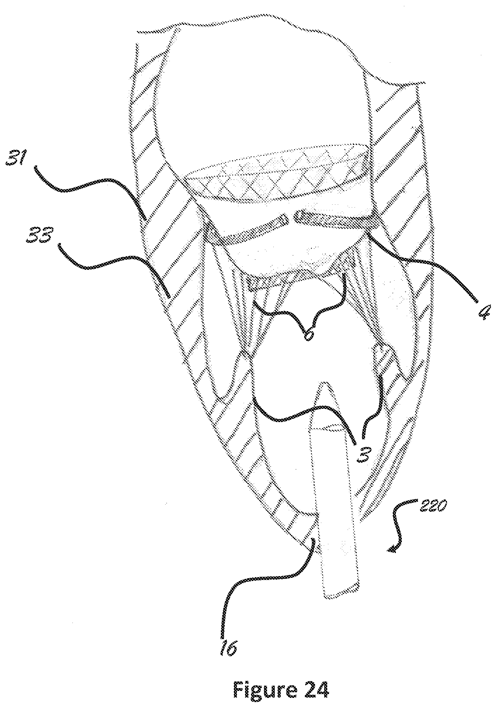

Figures 22A, 22B, 23, and 24 show an example of a trans apical approach for trans catheter implantation in the mitral position. In figures 22A and 22B, the catheter shaft 220 is advanced through the apex 16 of the heart and then through the native mitral valve to the left atrium. The stent 30 in this figure is crimped into the catheter shaft 220. The migration blocker rods are as well crimped in the shaft and can be crimped distally toward the atrium, proximally toward the entering point to the apex 16, or on top of the main frame 30. Figure 23 shows the deployment of the stent 30. The migration blocker rods 33 pass through the chordae 6 under the native commissures and circle the native leaflets. Then, a completion of the deployment results in clamping the native annulus and allowing the rods 33 to prevent migration and rocking. Figure 24 shows that the catheter is withdrawn backwards after completion of the deployment.

Exemplary aspects of the invention are set out below in paragraphs 1 to 16.
1. A prosthetic mitral valve assembly, comprising:
   a radially expandable stent including:
   an upper section anatomically configured to fit to a mitral valve annulus within a left atrium of a heart;
   a lower section coupled to the upper section and configured to fit within the mitral valve annulus; and
   migration blocker rods extending around at least a portion of a circumference of the lower section, the migration blocker rods to prevent axial movement of the stent with respect to the mitral valve annulus;
   and a replacement valve coupled to the stent.
2. The prosthetic mitral valve assembly of paragraph 1, wherein the radially expandable stent is configured to expand into clamping the mitral valve annulus by expanding from both sides of the mitral valve annulus, and wherein the migration blocker rods are behind native leaflets in a left ventricle and the upper section is above the mitral valve annulus in a left atrium.
3. The prosthetic mitral valve assembly of paragraph 1, wherein the replacement valve comprises a bicuspid or tricuspid valve.
4. The prosthetic mitral valve assembly of paragraph 1, wherein the migration blocker rods comprise two or more migration blocker rods that are configured to lock together during implantation.
5. The prosthetic mitral valve assembly of paragraph 1, wherein the migration blocker rods comprise two or more migration blocker rods configured to lock into the upper section through the mitral valve annulus.
6. The prosthetic mitral valve assembly of paragraph 1, wherein the migration blocker rods comprise two or more migration blocker rods comprising barbs to prevent rocking.
7. A prosthesis for securing a percutaneously implantable replacement valve in a heart,
   comprising:
   a radially expandable inflow section configured, in a deployed configuration, to be implanted within an atrium of a heart adjacent a native valve annulus of a heart valve, the inflow section including a proximal opening and a distal opening, the proximal opening having a greater circumference than that of the distal opening such that the inflow section is tapered;
   a radially expandable outflow section coupled to the distal opening of the inflow section, the outflow section configured, in a deployed configuration, to be implanted through the native valve annulus and at least partially within a ventricle of the heart; and
   two or more migration blocker rods extending circumferentially around at least a portion of the outflow section, a gap between the two or more migration blockers and the outflow section configured to hold native leaflets of the heart valve;
   wherein, in a contracted configuration, the prosthesis is configured to be implanted through a catheter into the heart; and
   wherein, in an expanded configuration, the tapered shape of the inflow section in the atrium
   cooperates with the two or more migration blockers in the ventricle to hold the prosthesis against the native valve annulus.
8. The prosthesis of paragraph 7, wherein, in the expanded configuration, the proximal opening of the inflow section, the distal opening of the inflow section, and a circumference of the outflow section are each substantially circular.
9. The prosthesis of paragraph 7, wherein, in the expanded configuration, the proximal opening of the inflow section is elliptical, and the distal opening of the inflow section and a circumference of the outflow section are each substantially circular.
10. The prosthesis of paragraph 7, wherein, in the expanded configuration, the proximal opening of the inflow section, the distal opening of the inflow section, and a circumference of the outflow section are each elliptical.
11. The prosthesis of paragraph 7, wherein, in the expanded configuration, the proximal opening of the inflow section is substantially circular, and the distal opening of the inflow section and a circumference of the outflow section are each elliptical.
12. The prosthesis of paragraph 7, wherein the inflow section comprises a shape memory material, and wherein the outflow section comprises one or more rows of expandable struts.
13. The prosthesis of paragraph 7, wherein respective ends of the two or more migration blocker rods are configured to lock together during deployment.
14. The prosthesis of paragraph 7, wherein the two or more migration blocker rods comprise barbs configured to extend, in the expanded configuration, through the native valve annulus and lock into the inflow section.
15. The prosthesis of paragraph 7, wherein the inflow section and the outflow section are separable from one another, the prosthetic valve assembly further comprising a plurality of attachment members to removably couple the outflow section to the inflow section.
16. The prosthesis of paragraph 7, wherein the inflow section is configured to secure a replacement valve.

It will be understood by those having skill in the art that many changes may be made to the details of the above-described embodiments without departing from the underlying principles of the invention. The scope of the present invention should, therefore, be determined only by the following claims.

## Claims

1. A prosthetic mitral valve assembly, comprising:
a radially expandable stent (30) including:
an upper section (31) having a tapered shape anatomically configured to fit to a mitral valve annulus within a left atrium (8) of a heart;
a lower section (32) coupled to the upper section (31) and configured to fit within the mitral valve annulus; and
a plurality of migration blocker rods (33), each migration blocker extending around at least a portion of a circumference of the lower section, the plurality of migration blocker rods configured to pass through a chordae under native commissures and to circle native leaflets to prevent axial movement of the stent with respect to the mitral valve annulus; and
a replacement valve coupled to the stent (30).

2. The prosthetic mitral valve assembly of claim 1, wherein the radially expandable stent (30) is configured to expand into clamping the mitral valve annulus by expanding from both sides of the mitral valve annulus, and wherein the migration blocker rods (33) are behind native leaflets in a left ventricle and the upper section is above the mitral valve annulus in a left atrium.

3. The prosthetic mitral valve assembly of claim 1, wherein the replacement valve comprises a bicuspid or tricuspid valve (52).

4. The prosthetic mitral valve assembly of claim 1, wherein the migration blocker rods comprise two or more migration blocker rods (33) that are configured to lock together during implantation.

5. The prosthetic mitral valve assembly of claim 1, wherein the migration blocker rods comprise two or more migration blocker rods (33) configured to lock into the upper section through the mitral valve annulus.

6. The prosthetic mitral valve assembly of clam 1, wherein the migration blocker rods (33) comprise two or more migration blocker rods comprising barbs to prevent rocking.

7. A prosthesis for securing a percutaneously implantable replacement valve in a heart, comprising:
a radially expandable inflow section (31) configured, in a deployed configuration, to be implanted within an atrium of a heart adjacent a native valve annulus of a heart valve, the inflow section (31) including a proximal opening and a distal opening, the proximal opening having a greater circumference than that of the distal opening such that the inflow section (31) is tapered;
a radially expandable outflow section (32) coupled to the distal opening of the inflow section (31), the outflow section (32) configured, in a deployed configuration, to be implanted through the native valve annulus and at least partially within a ventricle of the heart; and
two or more migration blocker rods (33) extending circumferentially around at least a portion of the outflow section (32) and configured to pass through a chordae to circle native leaflets, a gap between the two or more migration blocker rods (33) and the outflow section (32) configured to hold the native leaflets of the heart valve;
wherein, in a contracted configuration, the prosthesis is configured to be implanted through a catheter into the heart; and
wherein, in an expanded configuration, the tapered shape of the inflow section (31) in the atrium cooperates with the two or more migration blocker rods (33) in the ventricle to hold the prosthesis against the native valve annulus.

8. The prosthesis of claim 7, wherein, in the expanded configuration, the proximal opening of the inflow section (31), the distal opening of the inflow section (31), and a circumference of the outflow section (32) are each substantially circular.

9. The prosthesis of claim 7, wherein, in the expanded configuration, the proximal opening of the inflow section (31) is elliptical, and the distal opening of the inflow section (31) and a circumference of the outflow section (32) are each substantially circular.

10. The prosthesis of claim 7, wherein, in the expanded configuration, the proximal opening of the inflow section (31), the distal opening of the inflow section (31), and a circumference of the outflow section (32) are each elliptical.

11. The prosthesis of claim 7, wherein, in the expanded configuration, the proximal opening of the inflow section (31) is substantially circular, and the distal opening of the inflow section and a circumference of the outflow section (32) are each elliptical.

12. The prosthesis of claim 7, wherein the inflow section (31) comprises a shape memory material, and wherein the outflow section (32) comprises one or more rows of expandable struts.

13. The prosthesis of claim 7, wherein the two or more migration blocker rods (33) comprise barbs (120) configured to extend, in the expanded configuration, through the native valve annulus and lock into the inflow section (31).

14. The prosthesis of claim 7, wherein the inflow section (31) and the outflow section (32) are separable from one another, the prosthetic valve assembly further comprising a plurality of attachment members to removably couple the outflow section (32) to the inflow section (31).

15. The prosthesis of claim 7, wherein the inflow section (31) is configured to secure a replacement valve.
